# EUROPEAN PATENT APPLICATION

(11) **EP 2 727 956 A1**
(43) Date of publication of application: **07.05.2014**
(21) Application number: 12804328.8
(22) Date of filing: 26.06.2012
(51) Int. Cl.: C08L 5/08, B82B 1/00, A61L 27/40, A61L 27/50

(54) **BIOCOMPOSITE FOR FACILITATING ORGANIC TISSUE RECOVERY**

(30) Priority: 29.06.2011 BR PI1102686
(71) Applicant: Blay, Alberto, 01250-040 São Paulo, SP (BR); Awad Shibli, Jamil, Guarulhos - SP 07115-000 (BR); Tunchel, Samy, CEP: 05012-000 São Paulo, SP (BR); Vinicius Lucas Ferreira, Marcus, Belo Horizonte - MG 31310-220 (BR)
(72) Inventor: BLAY, Alberto, 01250-040 São Paulo (BR)
(74) Representative: Banchetti, Marina
(86) International application number: PCT/BR2012/000219
(87) International publication number: WO 2013/000049

(57) **Abstract**

"BIOCOMPOSITE FOR ORGANIC TISSUE REGENERATION", which features a porous structure with high permeability, diffusivity and biocompatibility to support cell growth within this matrix. The biocomposite was created with chitosan dispersed in an acid solution (pH 5 to 6) and sonicated until it forms a homogenous gelatinous structure into which purified multiwall carbon nanotubes (MWCNT) are introduced. This mixture is sonicated to create a solid block platform.

## Description

This report concerns a patent for the invention of a biocomposite for the recovery of organic tissue, and more specifically a biocomposite with a porous structure featuring high permeability, diffusivity and biocompatibility to support cell growth within this matrix. These materials are of great interest to fields of medicine involving tissue regeneration and engineering.

As experts in this field are well aware, the bone structure to be created must have certain favorable factors, such as a surface that permits cell adhesion and growth; that does not present any component or product resulting from degradation that is toxic and consequently does not provoke inflammatory reactions; that also permits a three-dimensional structural presentation; that has porosity with considerable surface area for cellular-scaffold interaction, i.e., a material that acts as a framework for the cells and also offers a space for the regeneration of an extracellular matrix.

The chitosan consists primarily of repetitive units of β(1, 4)2-amino-2-deoxy-d-glucose formed by the deacetylation of chitin, which is a polysaccharide that is abundantly produced in the shells of crustaceans. Previous research has revealed that chitosan presents bacteriostatic¹⁻⁴ and hemostatic properties⁵⁻⁷. It also presents excellent biocompatibility⁸⁻⁹, biocompatibility (10-11), is enzymatically degradable¹⁰ into absorbable oligosaccharides, can form insoluble complexes with connective tissue components such as collagen and glycosaminoglycans¹¹⁻¹³, can be used to produce porous structural supports, films and particles¹⁴, and has cell agglutination and hemostatic functions. It is also easily accessible and low-cost. Chitosan has been widely used to create biomaterial applications in various fields of biomedicine, such as tissue regeneration, devices for the controlled delivery of pharmaceuticals and gel-based cell immobilization systems.

Furthermore, the properties of the porous chitosan matrices, such as its microstructure and resistance to mechanical force, can be changed by altering the concentration of chitosan, the freezing rate, the molecular mass and the deacetylation rate of the initial material, or by combining it with composites of nanometric structures. In this last modification of the chitosan, the product can be used for the controlled delivery of pharmacological agents, as well as for the production of structural supports for use in tissue engineering.

However, the production of a structural support for durable and functional tissue engineering, especially for cartilage and bone tissues, remains a challenge¹⁵⁻¹⁸.

The main objective is to develop supports that provide better mechanical and functional properties for a variety of tissue engineering applications by improving the properties of the natural polymers with the incorporation of carbon nanotubes (CNT) to make stronger biomaterial composites. The mechanical properties must be improved for a variety of applications in soft and hard tissues, such as blood vessels, cartilage and bone.

It is well known that the presence of multiwall carbon nanotubes (MWCNT) promote changes in the porosity of the platform. However, more experiments and research must be conducted to improve the control of this porosity.

Therefore, studies and experiments were conducted that determined that variations in the MWCNT concentration affect the mechanical properties of the platform or structure.

These studies and experiments allowed for confirming that the presence of MWCNT represents a significant increase in resistance in comparison with the resistance of pure chitosan.

On the other hand, the presence of a higher percentage of reactive amino groups distributed in the chitosan's polymeric matrix allows for innumerous chemical modifications, such as the immobilization of chelating agents, quaternization, carboxylation, acylation, sulfonation, amidation, the formation of polyelectrolytic complexes, etc. In addition to the chitosan, the MWCNT itself, which was also made functional using hydroxyl groups, can also be modified with reactive chemical groups, allowing for various interactions for pharmaceutical and gene delivery or for increasing the resibilidade of the structural support in tissue engineering applications

Therefore, one of the objectives of this invention is to create a biocomposite for organic tissue regeneration that presents a high degree of biocompatibility and bioeletrochemical characteristics that facilitate cell growth.

Another objective of this invention is to obtain a biocomposite for the recovery of organic tissue that reduces the time required for the creation of the bone regeneration block or platform in comparison with the time required for the conventional creation of this block by using a process that involves washing carbon nanotubes in distilled water with centrifugation.

Another objective of this invention is to create a biocomposite for organic tissue regeneration that represents a lower-cost and easier process for obtaining the bone regeneration block.

These and other objectives and advantages of this invention are achieved using a biocomposite for organic tissue regeneration consisting of the preparation of biocomposites containing chitosan and multiwall carbon nanotubes (MWCNT) using thermally induced phase separation.

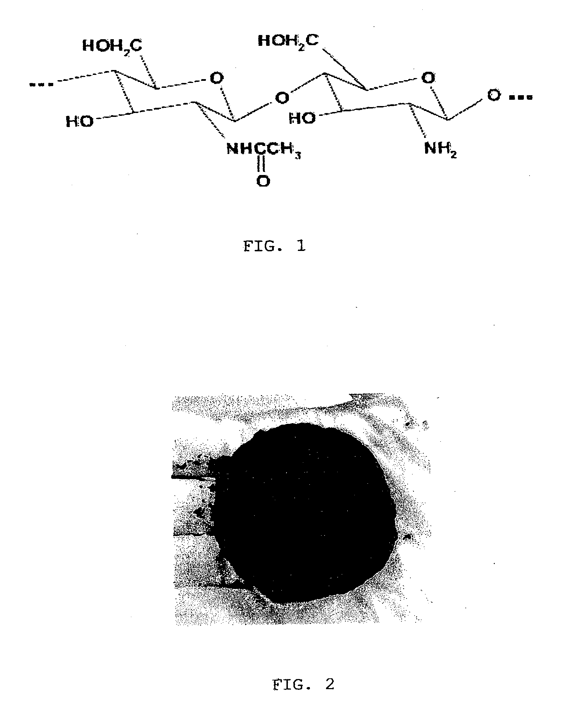

For this invention, the chitosan used, whose chemical formula is shown in Figure 1, has a degree of deacetylation of 85%. The carbon nanotubes (CNT) were treated in an HNO₃ solution (3-5M) and refluxed for 2 to 3 hours at 100° to 150°C. Next, the mixture was washed in distilled water and agitated at room temperature at 300rpm to eliminate impurities and excess acid in order to obtain a pH of 6 to 7. The product was dried at 100°-150°C for 2 to 3 hours and placed in varying quantities of multiwall carbon nanotubes (MWCNT) in 62mL of acetic acid (1 to 2M). The multiwall carbon nanotubes (MWCNT) were dispersed by sonication for 5 to 15 minutes at room temperature. Next, 2 to 3g of chitosan were added. The mixture was agitated at 1700rpm to obtain a homogenous mixture. The mixture was frozen at -40 to -60°C and soon afterwards the solvent was extracted using a lyophilization system for 40 to 50 hours. The composites were neutralized in an aqueous NaOH-CH₃CH₂CH₂OH solution (NaOH dissolved in 70 to 90% aqueous ethanol solution in a concentration of 0.5 to 2.0% NaOH by weight) at -20° to -40°C for 12 to 15 hours, immersed in ethyl alcohol for 12-15 hours, washed in distilled water until neutral and dried in a lyophilizer.

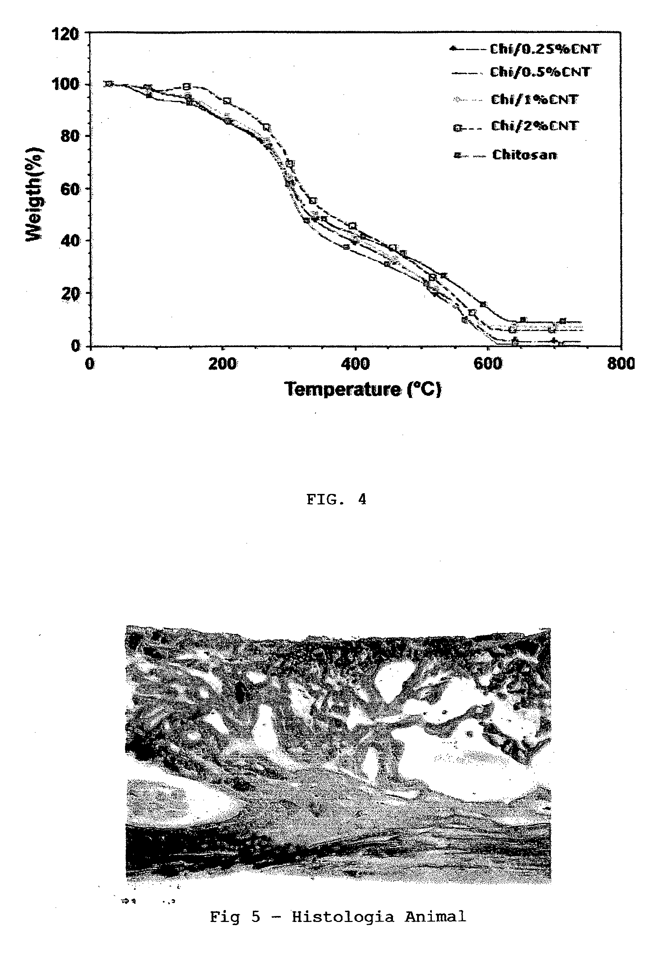

For the characterization of the composites, a simultaneous system utilizing thermogravimetric analysis/differential scanning calorimetry (TGA/DSC) was used to analyze the thermal stability of the composites and the chitosan (see Figure 4). The samples were cut into small pieces and heated at a constant rate of 10°C/min in a nitrogen atmosphere and an ambient temperature of between 310°C and 750°C with a flow of N₂ (rate of 20 mL/min). DSC TA was also used in a nitrogen atmosphere heated at a rate of 10°C/min.

These samples were initially heated to 120°C to eliminate their thermal history, chilled to-75°C and then finally reheated to 120°C. The infrared spectrums were obtained using an infrared spectrometer, with attenuated total reflection (ATR) using a Smiths Durasample IRII system with a diamond plate measuring 2mm in diameter.

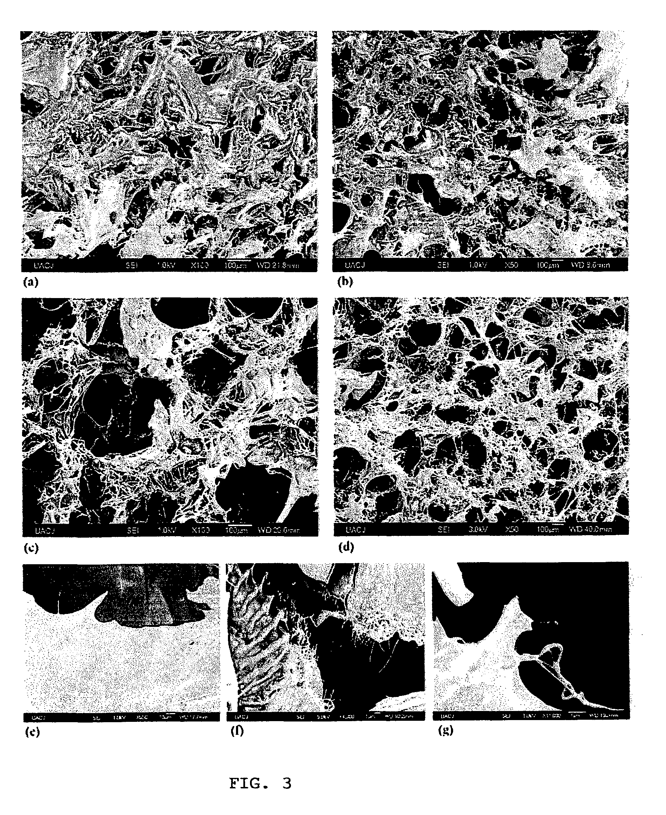

All samples were verified within the band of 550 to 4000 cm⁻¹. The morphologic characterization was conducted using a scanning electron microscope. For each sample force-deformation tests were conducted at 40°C at a deformation rate of 0.001mm/s. The porosity of the composite was determined using a liquid displacement method. Because it easily penetrates the pores and does not induce shrinkage or swelling, ethanol was used as the displacement liquid, as shown in Figure 3.

The biocomposite was synthesized using a chitosan solution containing 0.5 to 1.0g of chitosan with low and high molecular mass dissolved in 40 to 50mL of an aqueous solution of acetic acid (0.05 to 0.15M; pH 5.5 to 6.0) and was sonicated at room temperature to promote dispersion.

The multiwall carbon nanotubes (MWCNT) were first purified by immersion in hydrochloric acid (HCl) for two days in order to remove any impurities.

Soon after purification, they were carbolyxated using HNO₃ and HCl (20% to 37%) by reflux in microwaves for 15 to 30 minutes at a temperature of 110°C.

The product obtained was washed in distilled water in a centrifuge to obtain a solution with a pH of between 5 and 7.

After carboxylation and the subsequent washing, 90mg of multiwall carbon nanotubes (MWCNT) was agitated in a 1.5mL solution of chitosan of 1% to 2% with the mixture sonicated until it formed a solid block platform, as shown in Figure 2.

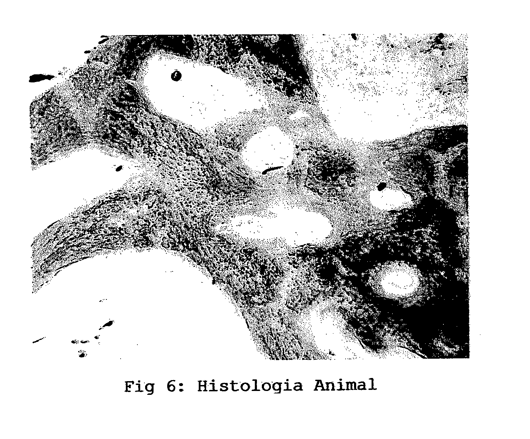

Figures 5 and 6 represent the animal histology with the use of the biocomposite. The blocks obtained from this process were subjected to liquid nitrogen for solidification with the subsequent introduction of these blocks in organisms with insufficient bone tissue to promote

## Claims

1. "BIOCOMPOSITE FOR ORGANIC TISSUE REGENERATION", **characterized by** the fact that it consists of the preparation of biocomposites containing chitosan and multiwall carbon nanotubes (MWCNT) using thermally induced phase separation.

2. "BIOCOMPOSITE FOR ORGANIC TISSUE REGENERATION", in accordance with Claim 1, **characterized by** the fact that chitosan presents a high degree of deacetylation of 85%; with the carbon nanotubes (CNT) refluxed in an HNO₃ solution (3 to 5M) for 2 to 3 hours at 100° to 150°C, with the mixture then washed in distilled water and agitated at room temperature at 300rpm to eliminate impurities and excess acid until a pH of 6 to 7 is obtained, with the product dried at 100° to 150°C for 2 to 3 hours and placed in varying quantities of multiwall carbon nanotubes (MWCNT) in 62mL of acetic acid (1 to 2M), with the multiwall carbon nanotubes (MWCNT) dispersed by sonication for 5 to 15 minutes at room temperature, to which 2 to 3g of chitosan is added, with the mixture agitated at 1700rpm to obtain a homogenous mixture, with this mixture frozen at -40 to -60°C and the solvent then extracted using a lyophilization system for 40 to 50 hours.

3. "BIOCOMPOSITE FOR ORGANIC TISSUE REGENERATION", in accordance with Claim 2, **characterized by** the fact that the composites were neutralized in an aqueous NaOH-CH₃CH₂CH₂OH solution (NaOH dissolved in a 70 to 90% aqueous ethanol solution in a concentration of 0.5 to 2.0% NaOH by weight) at-20° to -40°C for 12 hours, immersed in ethyl alcohol for 12 hours, washed in distilled water until neutral and dried in a lyophilizer.

4. "BIOCOMPOSITE FOR ORGANIC TISSUE REGENERATION", in accordance with Claim 2 or 3, **characterized by** the fact that the biocomposite was synthesized using a chitosan solution containing 0.5 to 1.0g of chitosan with low and high molecular mass dissolved in 40 to 50mL of an aqueous solution of acetic acid (0.05 to 0.15M; pH 5.5 to 6.0) and sonicated at room temperature to promote dispersion; with the multiwall carbon nanotubes (MWCNT) first purified by immersion in hydrochloric acid (HCl) for two days in order to remove any impurities; with carboxylation soon after purification using HNO₃ and HCl (20% to 37%) by reflux in microwaves for 15 to 30 minutes at a temperature of 110°C; with the product obtained then washed in distilled water in a centrifuge to obtain a solution with a pH of between 5 and 7; after carboxylation and subsequent washing, 90 to 100mg of multiwall carbon nanotubes (MWCNT) were agitated in a 1.5mL solution of chitosan of 1% to 2% with the mixture sonicated until it formed a solid block platform; with the blocks obtained from this process subjected to liquid nitrogen for solidification with the subsequent introduction of these blocks in organisms with insufficient bone tissue to promote tissue regeneration.
